# EUROPÄISCHE PATENTSCHRIFT

(11) **EP 1 928 436 B1**
(45) Veröffentlichungstag und Bekanntmachung des Hinweises auf die Patenterteilung: **04.07.2012**
(21) Anmeldenummer: 06777070.1
(22) Anmeldetag: 25.08.2006
(51) Int. Cl.: A61K 9/70, A61K 31/485

(54) **APPLIKATIONSSYSTEM FÜR EIN WIRKSTOFFHALTIGES PFLASTER UND WIRKSTOFFABGABEREGULIERUNGSMITTEL**
APPLICATION SYSTEM FOR A PLASTER CONTAINING AN ACTIVE INGREDIENT AND A CONTROLLED-RELEASE AGENT FOR SAID ACTIVE INGREDIENT
SYSTEME D'APPLICATION POUR UN PANSEMENT CONTENANT UN PRINCIPE ACTIF ET AGENT DE REGULATION DE LA LIBERATION DU PRINCIPE ACTIF

(30) Priorität: 09.09.2005 DE 202005014347 U
(43) Veröffentlichungstag der Anmeldung: 11.06.2008
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE)
(74) Vertreter: Kutzenberger, Helga
(86) Internationale Anmeldenummer: PCT/EP2006/008366
(87) Internationale Veröffentlichungsnummer: WO 2007/028513

(56) Entgegenhaltungen:
- EP-A2- 0 249 475
- WO-A2-02/41878
- WO-A2-2005/025549
- DE-A1- 19 733 981
- DE-A1- 19 900 645
- DE-A1-102004 020 463
- US-A- 4 917 688
- US-A1- 2003 125 659
- US-B1- 6 221 384

## Beschreibung

Die vorliegende Erfindung betrifft ein Applikationssystem (1) für ein Wirkstoffabgabesystem umfassend einen filmförmigen Streifen (2), ein damit lösbar verbundenes wirkstoffhaltiges Pflaster (3) und ein damit ebenfalls lösbar verbundenes, von dem wirkstoffhaltigen Pflaster separiert vorliegendes, teilbares Wirkstoffabgaberegulierungsmittel (6), wobei der filmförmige Streifen (2) eine Faltlinie (5) entsprechend seiner Breite und eine Breite aufweist, die mindestens der Breite des wirkstoffhaltigen Pflasters (3) und einer ggf. darüber hinaus gehenden Breite einer zur Abdeckung der Klebefläche des wirkstoffhaltigen Pflasters (3) verwendeten, ablösbaren Schutzfolie entspricht, und die Länge des Streifens (2) ein Mehrfaches der Länge des Pflasters (3) ist, und wobei das Wirkstoffabgaberegulierungsmittel (6) nach Entfernung der davon ablösbaren Schutzfolie auf der wirkstoffhaltigen Fläche (4) des Pflasters (3) nach Entfernung von dessen Schutzfolie durch Wiederzusammenfalten des Streifens (2) entlang der Faltlinie (5) dauerhaft positionierbar und das mit dem Wirkstoffabgaberegulierungsmittel versehene Pflaster direkt auf die Haut unter Entfernung des Streifens (2) applizierbar ist, wobei das Wirkstoffabgaberegulierungsmittel (6) vorgegebene Trennungsmöglichkeiten aufweist, über die es teilbar ist, und wobei das Wirkstoffabgaberegulierungsmittel (6) ein wirkstoffundurchlässiges Mittel ist oder ein die Wirkstoffabgabe retardierendes Mittel ist, das folgenden Schichtaufbau hat
a') eine außenliegende, lösbare Schutzschicht
b') eine ggf. die Wirkstoffabgabe retardierende Klebeschicht
c') ggf. eine die Wirkstoffabgabe ggf. weiter retardierende Schicht
d') und eine mit dem Streifen (2) lösbar verbundene weitere Klebeschicht.

Die topische oder systemische Abgabe eines Wirkstoffes über die Haut an einem menschlichen oder tierischen Körper erfolgt üblicherweise dadurch, dass über die Kontaktfläche zur Haut der Wirkstoff von der Haut aufgenommen wird.

Dabei wird üblicherweise über die gesamte Kontaktfläche des wirkstoffhaltigen Bereichs eines Pflasters der Wirkstoff so abgegeben.

Um eine individuelle Dosierung des Wirkstoffes bei einer solchen vorzugsweise transdermalen Applikation zu erreichen, muss bei den bekannten wirkstoffhaltigen Matrixpflastern der wirkstoffhaltige Bereich eines Pflasters, z. B. durch Zerschneiden verkleinert werden. Dies führt meist zu Ungenauigkeiten in der Dosierung. Eine individuelle Dosierung aus Pflastern, die den Wirkstoffe in einem Reservoir vorliegen haben, ist auf diese Weise nicht möglich.

Dies gilt auch für die in US 2003/0125659 beschriebene individuelle Dosierungsmöglichkeit von wirkstoffhaltigen Pflastern, die den Wirkstoff in einer Matrix eingebettet enthalten. Ein Nachteil der dort beschriebenen

Dosierungssysteme ist, dass die Dosierung nur von Pflastern mit einer bestimmten vorgegebenen Breite, die dem Auslaß der Dosierungsvorrichtung entspricht, geeignet ist. Eine entsprechende Verwendung der auf dem Markt erhältlichen unterschiedlichen wirkstoffhaltigen Matrixpflaster mit beliebiger Gestaltung ist nach diesem bekannten Dosierungssystem nicht möglich.

Weiterhin ist aus DE 197 33 981 eine begrenzte individuelle Dosierung eines transdermalen, therapeutischen Systems bekannt, indem ein bestimmter wirkstoffhaltiger Bereich eines Matrixpflasters mit einer für den Wirkstoff undurchlässigen Deckschicht bedeckt wird. Das System sieht nur eine einmalige Möglichkeit zur Verminderung der individuellen Dosis der transdermalen Wirkstoffabgabe vor.

Ein Wirkstoffabgabesystem für wirkstoffhaltige Matrix- oder Reservoir-Pflaster mit beliebiger Gestaltung, das eine individuelle, an den Patienten angepasste z. B. dem Körpergewicht entsprechende Dosierung des Wirkstoffes erlaubt, ist in der deutschen Patentanmeldung 10 2004 020 463.2 offenbart. Gemäß dieser Offenbarung kann mit Hilfe eines einzigen Wirkstoffabgaberegullerungsmittels eine unterschiedlich große Verminderung der Dosierung eingestellt und/oder eine Verabreichung des Wirkstoffes mit einer individuellen, an die Bedürfnisse des Patienten angepasste Freisetzung des Wirkstoffes erzielt werden.

Weiterhin ist als Wirkstoffabgabesystem zur systemischen oder topischen Abgabe eines Wirkstoffes durch und/oder an die Haut eines menschlichen oder tierischen Organismus ist auch ein System beschrieben, das ein wirkstoffhaltiges Pflaster und ein Wirkstoffabgaberegulierungsmittel umfasst. Vorzugsweise ist dieses Mittel ein gegebenenfalls teilbares, wirkstoffundurchlässiges oder ein gegebenenfalls teilbares, die Wirkstoffabgabe retardierendes Wirkstoffabgaberegulierungsmittel, das von dem Pflaster separiert vorliegt.

Solche Wirkstoffabgabesysteme sind nicht für alle Patienten einfach zu handhaben, da, um eine exakte Dosierung des Wirkstoffes zu erzielen, das Wirkstoffabgaberegulierungsmittel exakt auf der wirkstoffhaltigen Fläche des Pflasters in dem geforderten Ausmaß platziert werden muss. Da u. a. zu diesem Zweck auch die Klebeschicht des Wirkstoffabgaberegulierungsmittels freigelegt werden muss, kann dies leicht zu einem Vergeben des Mittels miteinander bzw. zumindest teilweise auf nicht wirkstoffhaltigen Arealen des Pflasters durch unsachgemäße Handhabung des Wirkstoffabgaberegulierungsmittels führen.

US 4 917 688 offenbart ein Bandagesystem zur transdermalen Abgabe eines Wirkstoffes, bei dem die Dosis an abgegebenem Wirkstoff angepasst werden kann.

WO 2005/025549 und EP 0 249 475 offenbaren faltbare Applikationssysteme für ein transdermales Wirkstoffabgabesystem.

Aufgabe der vorliegenden Erfindung war es, ein Applikationssystem für Wirkstoffabgabesysteme umfassend ein wirkstoffhaltiges Pflaster und ein Wirkstoffabgaberegulierungsmittel zur Verfügung zu stellen, das neben der Vermeidung einer Fehlplatzierung des Wirkstoffabgaberegulierungsmittels auf der wirkstoffhaltigen Schicht des Pflasters und einer erleichterten Handhabung für Patienten bzw. das Personal auch eine individuell an den Patienten angepasste Wirkstofffreisetzung ermöglicht.

Diese Aufgabe wird durch das zur Verfügung stellen des erfindungsgemäßen Applikationssystems gemäß Ansprüchen 1-15 gelöst.

Der filmförmige Streifen (Applikationsstreifen) ist vorzugsweise aus transparenter Kunststofffolie, die flexibel genug ist, gefaltet zu werden.

Vorzugsweise ist zumindest die Oberfläche des Applikationsstreifens, auf der das Wirkstoffabgaberegulierungsmittel fixiert ist, so ausgerüstet, dass sie Release-Eigenschaften aufweist.

Das erfindungsgemäße Applikationssystem weist das wirkstoffhaltige Pflaster und das Wirkstoffabgaberegulierungsmittel auf derselben Oberfläche des Applikationsstreifens auf. Dabei sind diese beiden Elemente in einem solchen Abstand von der Faltlinie des Applikationsstreifens positioniert, dass bei einer Faltung entlang der Faltlinie des Applikationsstreifens das Wirkstoffabgaberegulierungsmittel auf der wirkstoffhaltigen Fläche des Pflasters positioniert wird.

Das erfindungsgemäße Applikationssystem zeichnet sich weiterhin dadurch aus, dass die beiden Teile des Applikationsstreifens, die über die Faltlinie gegeneinander faltbar sind bzw. vor der Applikation des transdermalen Systems gegeneinander gefaltet werden, zumindest eine so unterschiedliche Länge aufweisen, dass ein Teil des Applikationsstreifens, der mit dem Wirkstoffabgaberegulierungsmittel lösbar verbunden ist, von der Faltlinie ausgehend zumindest die gesamte des Wirkstoffabgaberegulierungsmittels deckt.

In einer weiteren Ausführungsform des Applikationssystems können die beiden Teile des Applikationsstreifens, die über die Faltlinie gegeneinander faltbar sind, eine identische Länge aufweisen.

Die Breite des Applikationsstreifens entspricht mindestens der Gesamtbreite des wirkstoffhaltigen Pflasters, wobei der Applikationsstreifen vorzugsweise um mindestens 10% breiter ist als das wirkstoffhaltige Pflaster, um so die Positionierung des wirkstoffhaltigen Pflasters auf dem Applikationsstreifen zu erleichtem.

Das erfindungsgemäße Applikationssystem zeichnet sich dadurch aus, dass sowohl das Wirkstoffabgaberegulierungsmittel als auch das wirkstoffhaltige Pflaster mit einer ablösbaren Schutzfolie bedeckt sind. Diese ablösbare Schutzfolie weist vorzugsweise eine Ablösehilfe auf, die vorzugsweise in Form einer Schwächungslinie oder Trennlinie in der Schutzfolie vorliegt. Sofern eine Trennlinie in der Schutzfolie vorliegt, ist der mit dem übrigen Teil der Schutzfolie nicht verbundene Teil der Schutzfolie als Ablösehilfe gedacht. Die Schwächungslinie bzw. Trennlinie in der Schutzfolie ist vorzugsweise parallel zur Faltlinie des Applikationsstreifens angebracht. Es ist auch möglich, die Schutzfolie des wirkstoffhaltigen Pflasters und die Schutzfolie des Wirkstoffabgaberegulierungsmittels aus unterschiedlichen Materialien herzustellen.

Wie bereits ausgeführt, kann das Wirkstoffabgaberegulierungsmittel so ausgerüstet sein, dass es wirkstoffundurchlässig ist oder für die Wirkstoffabgabe regulierend, d. h. retardierend wirkt.

Vorzugsweise weist das gegebenenfalls teilbare, wirkstoffundurchlässige. Wirkstoffabgaberegulierungsmittel folgenden Schichtaufbau auf:
a) eine außen liegende lösbare Schutzschicht
b) eine Klebeschicht
c) eine Wirkstoffsperrschicht
d) eine weitere Klebeschicht, die mit dem Applikationsstreifen lösbar verbunden ist.

Diese weitere Klebeschicht dient zum Verbinden mit der Haut des Patienten.

Nach dem Ablösen der außen liegenden, lösbaren Schutzschicht sowohl von dem Pflaster als auch dem Wirkstoffabgaberegulierungsmittel wird durch Zusammenklappen des Applikationsstreifens der wirkstoffhaltige Bereich des Pflasters mit Hilfe des Wirkstoffabgaberegulierungsmittels im gewünschten Ausmaß teilweise abgedeckt. Durch Gegendruck und durch die Ablösehilfe der Schutzschicht wird verhindert, dass sich das Pflaster und/oder das Wirkstoffabgaberegulierungsmittel beim Ablösen der Schutzfolie gegebenenfalls auch vom Applikationsstreifen löst. Sofern das wirkstoffundurchlässige Wirkstoffabgaberegulierungsmittel teilbar ist, d. h. eine Perforationslinie oder Schwächungslinie durch das gesamte Wirkstoffabgaberegulierungsmittel enthält, kann vor Entfernung der äußeren, lösbaren Schutzschicht a) durch Faltung des Applikationsstreifens im Bereich dieser Schwächungslinie das Wirkstoffabgaberegulierungsmittel geteilt und der so abgeteilte Teil des Wirkstoffabgaberegulierungsmittels durch Abziehen von dem Applikationsstreifen insgesamt entfernt werden. Erst dann wird sowohl von dem wirkstoffhaltigen Pflaster als auch von dem Wirkstoffabgaberegulierungsmittel die außen liegende, lösbare Schutzschicht entfernt und die beiden Elemente durch Faltung des Applikationsstreifens entlang der Faltlinie und Zusammenklappen der beiden Teile des Applikationsstreifens aufeinander gebracht.

In einer bevorzugten Ausführungsform weist das wirkstoffundurchlässige Wirkstoffabgaberegulierungsmittel aber bereits das Ausmaß entsprechend dem wirkstoffhaltigen Bereich des Pflasters auf, der zur Dosierung der Wirkstoffabgabe, d. h. zur Erstellung einer verminderten Dosierung des Wirkstoffes, abgedeckt werden soll. Dazu wird die Größe des wirkstoffundurchlässigen Wirkstoffabgaberegulierungsmittels so geregelt, dass jeweils 10 bis 90% der wirkstoffhaltigen Fläche des wirkstoffhaltigen Pflasters abgedeckt werden.

Alternativ kann das Wirkstoffabgaberegulierungsmittel ein gegebenenfalls teilbares, die Wirkstoffabgabe nur retardierendes Wirkstoffabgaberegulierungsmittel sein. Vorzugsweise ist dieses Wirkstoffabgaberegulierungsmittel mehrschichtig und umfasst folgenden Schichtaufbau:
a') eine außenliegende, lösbare Schutzschicht
b') eine gegebenenfalls die Wirkstoffabgabe retardierende Klebeschicht
c') die Wirkstoffabgabe gegebenenfalls weiter retardierende Schicht und
d') eine mit dem Applikationsstreifen lösbar verbundene weitere Klebeschicht.

In einer besonders bevorzugten Ausführungsform weist das die Wirkstoffabgabe retardierende mehrschichtige Wirkstoffabgaberegulierungsmittel folgenden Schichtaufbau auf:
a') eine außenliegende, lösbare Schutzschicht
b') eine Klebeschicht
c') eine die Wirkstoffabgabe retardierende Schicht und
d') eine weitere Klebeschicht, die mit dem Applikationsstreifen lösbar verbunden ist und bei der Applikation mit der Haut des Patienten verbunden wird.

Sofern die beiden Klebeschichten b') und d') des die Wirkstoffabgabe retardierenden Wirkstoffabgaberegulierungsmittels zusammen dick genug sind, können diese vereinigt unter Weglassung einer die Wirkstoffabgabe retardierenden Schicht c') als die retardierende Schicht funktionieren. Vorzugsweise weist dann ein solches gegebenenfalls teilbares, die Wirkstoffabgabe retardierendes Wirkstoffabgaberegulierungsmittel, eine außenliegende, lösbare Schutzschicht a)' und eine Klebeschicht b') und d'), die die Wirkstoffabgabe retardiert und mit dem Applikationsstreifen lösbar verbunden ist und auf der Haut des Patienten appliziert wird, auf.

Vor der Applikation auf die Haut des Patienten wird die Schutzfolie sowohl von dem Pflaster als auch von dem Wirkstoffabgaberegulierungsmittel, das eine Retardierung der Wirkstoffabgabe bewirkt, entfernt und das Wirkstoffabgaberegulierungsmittel durch Faltung des Applikationsstreifens entlang der Faltlinie des Wirkstoffabgaberegulierungsmittels auf dem wirkstoffhaltigen Bereich des Pflasters plaziert. Durch Gegendruck und durch die Ablösehilfe der Schutzfolie wird verhindert, dass sich das Pflaster und/oder das Wirkstoffabgaberegulierungsmittel beim Ablösen der Schutzfolie gegebenenfalls auch schon vom Applikationsstreifen löst.

Sofern das Wirkstoffabgaberegulierungsmittel mit einer die Wirkstoffabgabe retardierenden Schicht ausgerüstet und teilbar ist, wird vorzugsweise vor dem Ablösen der außen liegenden Schutzschicht.das Wirkstoffabgaberegulierungsmittel geteilt. Dies kann - wie vorstehend ausgeführt - entlang der für die Trennungsmöglichkeiten vorgesehenen Schwächungslinien, wie Perforationen bzw. Trennlinien durch das gesamte Wirkstoffabgaberegulierungsmittel, durch Faltung entlang solcher Trennlinien und Ablösen eines abgetrennten Teils des Wirkstoffabgaberegulierungs-mittels erfolgen. Erst dann wird die außen liegende Schutzfolie von dem Wirkstoffabgaberegulierungsmittel und von dem wirkstoffhaltigen Pflaster abgezogen und die beiden Teile des Applikationsstreifens entlang der Faltungslinie zum Aufbringen des Wirkstoffabgaberegulierungsmittels auf den wirkstoffhaltigen Bereich des Pflasters gefaltet.

Die Größe eines die Wirkstoffe retardierenden Wirkstoffabgaberegulierungsmittels kann bis zu 100% des wirkstoffhaltigen Bereichs eines Pflasters ausmachen. In einer bevorzugten Ausführungsform ist die Bedeckung 100%. Sofern die Bedeckung weniger als 100% ist, erhält man ein Pflaster mit einer unterschiedlich retardierten Abgabe des Wirkstoffes, d. h. einer retardierten und üblichen Wirkstoffabgaberate.

Sowohl das wirkstoffhaltige Pflaster als auch das Wirkstoffabgaberegulierungsmittel können jede beliebige Form, Größe und Farbgebung aufweisen. Beispielsweise können sie unabhängig voneinander eine runde, ovale, rechteckige oder eine andere unregelmäßige Form aufweisen. Vorzugsweise weist das Pflaster, insbesondere der wirkstoffhaltige Bereich des Pflasters und das Wirkstoffabgaberegulierungsmittel, zueinander passende, vorzugsweise identische, Formen auf.

Vorzugsweise können die voneinander trennbaren Teile eines teilbaren Wirkstoffabgaberegulierungsmittels eine unterschiedliche Markierung zur Unterscheidung aufweisen. Diese Markierung zur Unterscheidung kann eine unterschiedliche Färbung und/oder Codierung der voneinander abtrennbaren Teile sein.

Das Wirkstoffabgabesystem mit Applikationsstreifen eignet sich für die Applikation jedes applizierbaren Wirkstoffes. Vorzugsweise eignet sich das Wirkstoffabgabesystem zur transdermalen und/oder topischen Verabreichung von Wirkstoffen jeder Art, besonders bevorzugt von pharmazeutischen Wirkstoffen jeder Art. Insbesondere eignet sich das Pflaster zur transdermalen und/oder topischen Abgabe von wenigstens einem pharmazeutischen Wirkstoff aus der Gruppe umfassend Analgetica, Lokalanästhetica, Hormone, Kontrazeptiva, Impfstoffe, Immunmodulatoren, Antiallergica, Antihistaminica, Cardiaca, Antihypertonica, Psychopharmaca, Antirheumatica und Enzyme, bevorzugt zur Verabreichung wenigstens eines pharmazeutischen Wirkstoffes ausgewählt aus der Gruppe umfassend Narkotika, Opioide, Tranquillantien, vorzugsweise Benzodiazepine, Stimulantien und andere Betäubungsmittel.

Besonders bevorzugt eignet sich das erfindungsgemäße Applikationssystem zur Verabreichung mindestens eines transdermal verabreichbaren Opioids, Tranquillanz oder eines anderen Betäubungsmittels, das ausgewählt ist aus der Gruppe umfassend
N-{1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3H)-on (Bromazepam), 2-Brom-4-(2-chlorphenyl)-9-methyl-6H-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5H)-on (Cloxazolam), (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor 5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dextromethorphan, Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimepheptanol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-8-methoxy-17-methyl-6,14-*endo*-etheno-morphinan-3-ol (Etorphin), *N-*Ethyl-3-phenyl-8,9,10-trinorboman-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Levoxemacin, Lofentanil, 6-(2-Chlorphenyl)-2-(4-methyl-1-piperazinylmethylen)-8-nitro-2*H* imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3H)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-*a*]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-o-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-*a*][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,1Oα-tetrahydro-1-hydroxy-6,6-dimethyl-6*H*-dibenzo [*b*, *d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Nortevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis-trans*)-10-Chlor-2,3,7,11b-tetrahydro-2-methyl-11b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen, Papaveretum, 2-Imino-5-phenyl-4-oxazolidinon (Pemolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin; Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Premethadion, Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N*-phenylpropanamido)piperidino]propanoat} (Remifentanil), 5-*sec*-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1S,2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1RS, 3RS, 6RS)-6-Dimethylaminomethyl-1-(3-methoxy-phenyl)-cyclohexan-1,3-diol, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäre 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie deren entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt deren Hydrochloride.

Die Verbindungen (1 R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenylkyclohexanol oder deren stereoisomere Verbindungen oder deren physiologisch verträgliche Verbindungen, insbesondere deren Hydrochloride, deren Derivate, wie Ester, Ether oder Amide sowie Verfahren zu ihrer Herstellung sind beispielsweise aus EP-A-693 475 bzw. EP-A-780 369 bekannt.

Als transdermal zu verabreichende pharmazeutische Wirkstoffe werden vorzugsweise Opioide eingesetzt. Ganz besonders bevorzugt sind diese Wirkstoffe ausgewählt aus der Gruppe umfassend Morphin, Oxycodon, Buprenorphin und Fentanyl, deren Derivate, vorzugsweise Ester, Ether oder Amide, oder deren jeweils physiologisch verträglichen Verbindungen, vorzugsweise deren Salze oder Solvate, besonders bevorzugt deren Hydrochloride. Besonders bevorzugt ist der Einsatz der freien Base.

Bevorzugt liegt die Konzentration des Wirkstoffes bei seiner Sättigungskonzentration oder geringfügig darunter, da dadurch die Abgabe an die Haut gefördert wird. Diese Sättigungskonzentration kann durch Routineversuche ermittelt werden.

Die Wirkstoffsperrschicht des Wirkstoffabgaberegulierungsmittets besteht vorzugsweise aus einem Polymeren wie Polyester, z. B. Polyethylenterephthalat, Polyolefin, wie Polyethylen, Polypropylen oder Polybutylen, Polycarbonat, Polyethylenoxid, Polyurethan, Polystyrol, Polyamid, Polyimid, Polyvinylacetat, Polyvinylchlorid, Polyvinylidenchlorid, und/oder Copolymere aus z. B. Acrylonitril/Butadien/Styrol. Die Dicke der Schicht beträgt vorzugsweise 5 bis 25 µm.

Die die Wirkstoffabgabe retardierende Schicht eines Wirkstoffabgaberegulierungsmittels kann aus einem filmbildenden Polymeren ausgewählt aus der Gruppe umfassend Cellulose-Derivate, wie Ethylcellulose, Hydroxypropylcellulose oder Carboxymethylcellulose, Polyethylene, chlorierte Polyethylene, Polypropylene, Polyurethane, Polycarbonate, Polyacrylsäureester, Polyacrylate, Polymethacrylate, Polyvinylalkohole, Polyvinylchloride, Polyvinylidenchloride, Polyvinylpyrrolidone, Polyethylenterephthalate, Polytetrafluoroethylene, Ethylen/Propylen Copolymere, Ethylen/Ethylacrylat Copolymere. Ethylen/Vinylacetat Copolymere, Ethylen/Vinylalkohol Copolymere, Vinylchlorid/Vinylacetat Copolymere, Vinylpyrrolidon/Ethylen/Vinylacetat Copolymere, Kautschuke, gummiartige, synthetische Homo-, Co- oder Blockpolymere, Silikone, Silikon-Derivate und deren Mischungen bestehen.

Als eine die Wirkstoffabgabe retardierende Schicht kann vorzugsweise eine Schicht basierend auf einem Ethylen/Vinylacetat Copolymerisat, einem Polyacrylat oder eine aus den beiden Klebeschichten b') und d') kombinierte Schicht unter Wegfall einer separaten, die Wirkstoffabgabe retardierenden Schicht eingesetzt werden, die für die vorgegebene Zeit des Pflastergebrauchs, vorzugsweise für 3 bis 7 Tage die Wirkstoffabgabe retardiert. Übliche Wirkstoffreservoirmembrane können ebenfalls als retardierende Schicht eingesetzt werden.

Das wirkstoffhaltige Pflaster kann nach dem Reservoir- oder Matrix- System aufgebaut sein (Bauer K. H., Frömming K.-H., Führer C., Pharmazeutische Technologie, Seiten 381-383; Müller R. H., Hildebrand G. E., Pharmazeutische Technologie: Moderne Arzneiformen, Kapitel 8).

Gemäß dem Matrix-System kann das wirkstoffhaltige Pflaster vorzugsweise eine Trägerschicht, eine wirkstoffhaltige Schicht und eine Klebeschicht aufweisen, wobei die wirkstoffhaltige Schicht gleichzeitig die Klebeschicht sein kann, in der der Wirkstoff gelöst und/oder dispergiert in einer Matrix zusammen mit dem Klebstoff vorliegt. Das wirkstoffhaltige Pflaster weist eine außen liegende, lösbare Schutzschicht auf.

Als Klebstoffe für die Klebeschicht des Pflasters und des Wirkstoffabgaberegulierungsmittels werden vorzugsweise druckempfindliche Klebstoffe ("pressure-sensitive adhesives") eingesetzt. Beispielsweise eignen sich dafür Polymere wie Polyacrylate, Polyvinylether, Polyisobutylene (PIB), Styrol/Isopren- oder Butadien-/Styrol Copolymere oder Polyisopren Kautschuke. Weiterhin eignen sich Silikon-Klebstoffe, wie z. B. gegebenenfalls-vernetzte Polydimethylsiloxane. Ferner sind Kunststoffe auf Basis von Ester von Glycinen, Glycerin oder Pentaerythrol, oder von Kohlenwasserstoffen, wie Polyterpene geeignet. Klebstoffe auf Polyacrylatbasis werden durch Polymerisation von Acrylaten, Methacrylaten, Alkylacrylaten und/oder Alkylmethacrylaten, mit gegebenenfalls weiteren α, β- ungesättigten Monomeren, wie Acrylamid, Dimethylacrylamid, Dimethylaminoethylacrylat, Hydroxyethylacrylat, Hydroxypropylacrylat, Methoxyethylacrylat, Methoxyethylmethacrylat, Acrylnitril und/oder Vinylacetat, hergestellt. Mit diesen druckempfindlichen Klebstoffen kann auch die Trägerschicht bzw. Deckschicht des Pflasters zumindest punktuell, vorzugsweise vollflächig, mit dem Applikationsstreifen verbunden sein.

Die Klebeschichten des Pflasters und des Wirkstoffabgaberegulierungsmittels können auch Hautdurchdringungsverstärker, Füllstoffe (wie Zinkoxid oder Silika), Vernetzer, Antioxidationsmittel und/oder Lösungsmittel enthalten. Die Dicke der Klebeschichten beträgt vorzugsweise jeweils 3 bis 100 µm.

Die Trägerschicht bzw. Deckschicht des Pflasters ist vorzugsweise für die in der wirkstoffhaltigen Schicht und in der Klebeschicht enthaltenen Stoffen, insbesonders für den transdermal und/oder topisch abzugebenden Wirkstoff undurchlässig und inert, und kann auf Polymeren, wie Polyester, z. B. Polyethylenterephthalat; Polyolefinen, wie Polyethylenen, Polypropylenen oder Polybutylenen; Polycarbonaten; Polyethylenoxiden, Polyurethanen, Polystyrrolen, Polyamiden, Polyimiden, Polyvinylacetaten, Polyvinylchloriden, Polyvinylidenchloriden und/oder Copolymeren wie Acrylonitril/Butadie/Styrrol Copolymeren und/oder deren Mischungen gegebenenfalls enthaltend Papierfasern und/oder Textilfasern basieren, wobei die Fasern bei Bedarf metallisiert oder pigmentiert sein können. Die Trägerschicht bzw. Deckschicht des Pflasters kann auch aus einer Kombination aus Metallfolie und Polymerschicht bestehen. Die Dicke der Trägerschicht beträgt vorzugsweise 3 bis 100 µm.

Zur Fixierung des Pflasters auf dem Applikationsstreifen weist der Streifen zumindest im Randbereich des Pflasters an seiner Oberfläche eine Schicht aus druckempfindlichem Klebstoff-auf. Die Fixierung kann auch mit Hilfe eines Doppelklebestreifens erreicht werden.

Die wirkstoffhaltige Matrix-Schicht des Pflasters kann matrixbildende Polymere, Hautdurchdringungsverstärker, Lösungsvermittler, Vernetzer, Stabilisatoren, Emulgatoren, Konservierungsmittel, Verdickungsmittel und/oder weitere übliche Hilfsmittel enthalten.

Als matrixbildendes Polymeres wird vorzugsweise wenigstens ein filmbildendes Polymeres ausgewählt aus der Gruppe umfassend Hydroxypropylcellulose, Carboxymethylcellulose, Polyethylene, chlorierte Polyethylene, Polypropylene, Polyurethane, Polycarbonate, Polyacrylsäureester, Polyacrylate, Polymethacrylate, Polyvinylalkohole, Polyvinylchloride, Polyvinylidenchloride, Polyvinylpyrrolidone, Polyethylenterephthalate, Polytetrafluoroethylene, Ethylen/Propylen Copolymere, Ethylen/Ethylacrytat Copolymere, Ethylen/Vinylacetat Copolymere, Ethylen/Vinylalkohol Copolymere, Ethylen/Vinyloxyethanol Copolymere, Vinylchlorid/Vinylacetat Copolymere, Vinylpyrrolidon/Ethylen/Vinylacetat Copolymere, Kautschuke, gummiartige, synthetische Homo-, Co- oder Blockpolymere, Silikone, Silikon-Derivate, vorzugsweise Siloxan/Methacrylat Copolymere, Cellulose-Derivate, vorzugsweise Ethylcellulose oder Celluloseether und deren Mischungen eingesetzt. Wenn die wirkstoffhaltige Schicht gleichzeitig die Klebeschicht ist, enthält sie vorzugsweise neben wenigstens einem der aufgezählten Polymeren zumindest einen der vorstehend aufgeführten Klebstoffe.

Als Lösungsvermittler können N-methyl-2-pyrrolidon, Laurylpyrrolidon, Triethanolamin, Triacetin, Diethylenglykol-monoethylether, Derivate von Fettsäuren oder Fettalkoholen, niedermolekulare, mehrwertige Alkohole wie beispielsweise Propylenglycol oder Glycerin und/oder tensidische Verbindungen verwendet werden.

Wenn das wirkstoffhaltige Pflaster nach dem Reservoir-System aufgebaut ist, kann die Reservoir-Membran aus inerten Polymeren wie z. B. Polyethylenen, Polypropylenen, Polyvinylacetaten, Polyamiden, EthylenNinylacetat Copolymeren und/oder Silikonen bestehen. Mit Hilfe der Reservoir-Membran kann aus dem Reservoir bereits eine kontrollierte Freisetzung des Wirkstoffes erzielt werden.

Die wirkstoffhaltige Matrix bzw. das wirkstoffhaltige Reservoir des Pflasters kann auch Lösungsmittel, wie z. B. Wasser, Ethanol, 1-Propanol, Isopropanol, einen niedermolekularen, mehrwertigen Alkohol, beispielsweise Propylenglycol oder Glycerin, oder einen Ester, wie Isopropylmyristat, tensidische Verbindungen oder deren Mischungen enthalten.

Als Stabilisatoren für die wirkstoffhaltige Matrix bzw. für den Inhalt des wirkstoffhaltigen Reservoirs können Antioxidantien, wie Vitamin E, Butylhydroxytoluol, Butylhydroxyanisol, Ascorbinsäure, Ascorbylpalmitat, und/oder Chelatbildner, wie z. B. Dinatriumethylendiamintetraessigsäure, Kalium- oder Natriumcitrat verwendet werden.

Die wirkstoffhaltige Matrix bzw. das wirkstoffhaltige Reservoir kann auch übliche Hautdurchdringungsverstärker enthalten.

Das Pflaster kann auch in einer oder mehreren Schichten wenigstens einen Weichmacher oder Hautdurchdringungsverstärker ausgewählt aus der Gruppe umfassend langkettige Alkohole, wie Dodecanol, Undecanol, Octanol, Ester von Carbonsäuren mit polyethoxylierten Alkoholen, Diester von aliphatischen Dicarbonsäuren, wie Adipinsäure, und mittelkettige Triglyceride von Caprylsäure und/oder Caprinsäure, Kokosfett, mehrwertige Alkohole, wie 1,2-Propandiol, Ester von mehrwertigen Alkoholen, wie Glycerin mit Lävulinsäure oder Caprylsäure, und veretherte mehrwertige Alkohole enthalten.

Die außen liegende, lösbare Schutzschicht des Pflasters und die außen liegende, lösbare Schutzschicht des Wirkstoffabgaberegulierungsmittels, die mit der Größe des Applikationsstreifens identisch sein kann und mit dem Pflaster, dem Wirkstoffabgaberegulierungsmittel und mit der damit nicht bedeckten Oberfläche des Applikationsstreifens lösbar verbunden sein kann, kann aus Polyethylen, Polyester, Polyethylenterephthalat, Polypropylen, Polysiloxan, Polyvinylchlorid oder Polyurethan und gegebenenfalls aus behandelten Papierfasern, wie z. B. Zellophan, bestehen und gegebenenfalls wenigstens eine Silikon-, Fluorsilikon- oder Fluor-Kohlenstoffbeschichtung aufweisen.

Die Herstellung des erfindungsgemäßen Wirkstoffabgaberegulierungsmittels bzw. des Pflaster kann nach bekannten Herstellungsverfahren erfolgen, die die Verfahrensschritte Laminieren, Coextrudieren, Stanzen, Delaminieren, Abwickeln, Schneiden, Wiederaufwickeln, Montieren und/oder Dosieren (Verpackungs-Rundschau 4/2002, 83-84) umfassen.

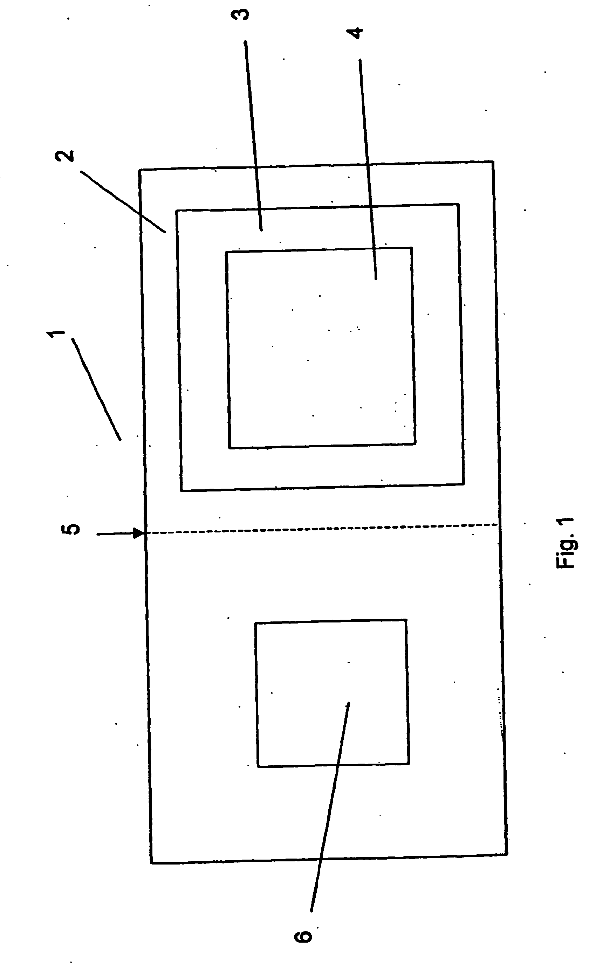

- **Figur 1**: zeigt ein erfindungsgemäßes Applikationssystem (1) für ein Wirkstoffabgabesystem umfassend einen filmförmigen Applikationsstreifen (2), ein damit lösbar verbundenes wirkstoffhaltiges Pflaster (3), das einen wirkstoffhaltigen Bereich (4) aufweist, und ein vom Pflaster separiert vorliegendes Wirkstoffabgaberegulierungsmittel (6), das über eine Faltlinie (5) des Applikationsstreifens nach Ablösen der Schutzfolie (nicht gezeigt) sowohl von dem wirkstoffhaltigen Pflaster als auch von dem Wirkstoffabgaberegulierungsmittel durch Faltung entlang der Faltlinie (5) mit dem wirkstoffhaltigen Bereich des Pflasters verbunden werden kann.

### Beispiel

### a) Herstellung eines Buprenorphin-haltigen Pflasters

1139 g einer 48 Gew.%igen Polyacrylatlösung eines selbstvernetzenden Acrylatcopolymeren aus 2-Ethylhexylacrylat, Vinylacetat, Acrylsäure (Lösungsmittel: Ethylacetat:Heptan:Isopropanol:Toluol:Acetylacetonat im Verhältnis von 37:26:26:4:1), 100 g Lävulinsäure, 150 g Oleylacetat, 100 g Polyvinylpyrrolidon, 150 g Ethanol, 200 g Ethylacetat und 100 g Buprenorphinbase wurden homogenisiert. Man rührte etwa zwei Stunden und kontrollierte visuell, ob alle Feststoffe gelöst waren. Man kontrollierte außerdem den Verdunstungsverlust durch Zurückwiegen und ergänzte gegebenenfalls den Lösungsmittelverlust durch Zugabe von Ethylacetat.

Als Deckschicht wurde eine 420 mm breite, transparente Polyesterfolie mit der vorstehend beschriebenen Mischung so beschichtet, dass das Flächengewicht der getrockneten Klebeschicht bei 80 g/m² lag.

Man entfernte die Lösungsmittel durch Trocknen mit erwärmter Luft, die über die feuchte Bahn geleitet wurde. Abschließend deckte man die wirkstoffhaltige Klebeschicht mit einer 15 µm dicken Polyesterfolie ab, die durch eine Silikonbehandlung wieder ablösbar war. Mit geeigneten Schneidewerkzeugen stanzte man eine für die vorgesehene Wirkstoffmenge entsprechende Fläche aus.

### b) Herstellung eines Wirkstoffabgaberegulierungsmitteis mit dem folgenden Schichtaufbau:

- eine außen liegende, lösbare Schutzschicht
- eine Klebeschicht
- eine Wirkstoffsperrschicht
- eine Klebeschicht
- eine zur Fixierung auf dem Applikationsstreifen lösbare Schutzschicht

Zur Herstellung des Wirkstoffabgaberegulierungsmittels wurde eine Polyethylenterephthalat-Folie mit einer Dicke von 75 µm als Wirkstoffsperrschicht in einem Ericsson-Filmziehgerät (der Fa. Ericsson GmbH & Co. KG, Hemer, Deutschland) eingespannt, mit der unter Beispiel 1 a) mit Ausnahme von Buprenorphin beschriebenen Mischung beschichtet und 2 Stunden getrocknet, wodurch man eine Klebeschicht mit einer Dicke von 90 µm erhielt. Diese Klebeschicht wurde mit einer einseitig silikonisierten Folie auf Polyethylenterephthalatbasis mit der silikonisierten Seite lösbar verbunden.

Auf der noch freien zweiten Oberfläche der Wirkstoffsperrschicht wurde der Vorgang wiederholt, so dass die Wirkstoffsperrschicht beidseitig eine Klebeschicht mit einer Dicke von 90 µm und einer damit lösbar verbundenen Schutzschicht aufwies.

Mit Hilfe eines geeigneten Stanzwerkzeuges wurde das Wirkstoffabgaberegulierungsmittel mit einer Größe gemäß der halben Größe der nach Bespiel 1 a) hergestellten Pflaster ausgestanzt.
c) Auf einem Streifen aus transparenter Polyesterfolie, der entlang einer Faltlinie in zwei gleich große Teile faltbar ist, wurde zu beiden Seiten der Faltlinie auf derselben Oberfläche einerseits das nach 1 a) hergestellte Pflaster und andererseits das nach 1 b) hergestellte Wirkstoffabgaberegulierungsmittel fixiert. Die Anordnung der beiden Elemente des transdermalen Abgabesystems entspricht der in Figur 1 gezeigten. Zur Fixierung des Pflasters wurde der Applikationsstreifen in dem Bereich des zu fixierenden Pflasters mit einer Schicht aus druckempfindlichem Klebstoff ausgerüstet. Die Fixierung des Wirkstoffabgaberegulierungsmittels erfolgte nach Ablösung einer der außen liegenden, lösbaren Schutzschichten des Mittels durch die dadurch freiwerdende Klebeschicht:

Vorzugsweise können die jeweils verbleibenden außen liegenden, lösbaren Schutzschichten vom Pflaster und dem Wirkstoffabgaberegulierungsmittel auch noch abgelöst werden und die gesamte, mit dem Pflaster und dem Mittel versehene Oberfläche des Applikationsstreifens mit einer lösbaren, metallisierten Schutzfolie mit Ablösehilfe bedeckt werden.

Vorzugsweise kann das erfindungsgemäße Applikationssystem über die Faltlinie gefaltet und vorzugsweise steril und/oder gasdicht verpackt auf den Markt gebracht werden.

## Patentansprüche

1. Applikationssystem (1) für ein Wirkstoffabgabesystem umfassend einen filmförmigen Streifen (2), ein damit lösbar verbundenes wirkstoffhaltiges Pflaster (3) und ein damit ebenfalls lösbar verbundenes, von dem wirkstoffhaltigen Pflaster separiert vorliegendes, teilbares Wirkstoffabgaberegulierungsmittel (6), wobei der filmförmige Streifen (2) eine Faltlinie (5) entsprechend seiner Breite und eine Breite aufweist, die mindestens der Breite des wirkstoffhaltigen Pflasters (3) und einer ggf. darüber hinaus gehenden Breite einer zur Abdeckung der Klebefläche des wirkstoffhaltigen Pflasters (3) verwendeten, ablösbaren Schutzfolie entspricht, und die Länge des Streifens (2) ein Mehrfaches der Länge des Pflasters (3) ist, und wobei das Wirkstoffabgaberegulierungsmittel (6) nach Entfernung der davon ablösbaren Schutzfolie auf der wirkstoffhaltigen Fläche (4) des Pflasters (3) nach Entfernung von dessen Schutzfolie durch Wiederzusammenfalten des Streifens (2) entlang der Faltlinie (5) dauerhaft positionierbar und das mit dem Wirkstoffabgaberegulierungsmittel versehene Pflaster direkt auf die Haut unter Entfernung des Streifens (2) applizierbar ist, wobei das Wirkstoffabgaberegulierungsmittel (6) vorgegebene Trennungsmöglichkeiten aufweist, über die es teilbar ist, und wobei das Wirkstoffabgaberegulierungsmittel (6) ein wirkstoffundurchlässiges Mittel ist oder ein die Wirkstoffabgabe retardierendes Mittel ist, das folgenden Schichtaufbau hat:
a') eine außenliegende, lösbare Schutzschicht
b') eine ggf. die Wirkstoffabgabe retardierende Klebeschicht
c') ggf. eine die Wirkstoffabgabe ggf. weiter retardierende Schicht
d') und eine mit dem Streifen (2) lösbar verbundene weitere Klebeschicht.

2. Applikationssystem nach Anspruch 1, **dadurch gekennzeichnet, dass** der Streifen aus einer vorzugsweise transparenten Kunststoffolie besteht.

3. Applikationssystem nach Anspruch 1 oder 2, **dadurch gekennzeichnet, dass** zumindest die Oberfläche des Streifens (2), auf der das Wirkstoffabgaberegulierungsmittel befestigt ist, Release-Eigenschaften aufweist.

4. Applikationssystem nach einem der Ansprüche 1 bis 3, **dadurch gekennzeichnet, dass** das wirkstoffhaltige Pflasters und das Wirkstoffabgaberegulierungsmittel auf derselben Oberfläche des Streifens und jeweils in einem solchen Abstand von der Faltlinie (5) positioniert sind, dass bei einer Faltung entlang der Faltlinie (5) des Streifens das Wirkstoffabgaberegulierungsmittel auf der wirkstoffhaltigen Fläche des Pflasters (4) positionierbar ist.

5. Applikationssystem nach einem der Ansprüche 1 bis 4, **dadurch gekennzeichnet, dass** sowohl das Wirkstoffabgaberegulierungsmittel (6) als auch das wirkstoffhaltige Pflaster (3) mit einer außen liegenden, ablösbaren Schutzfolie bedeckt sind, die vorzugsweise eine Ablösehilfe aufweist.

6. Applikationssystem nach Anspruch 5, **dadurch gekennzeichnet, dass** die Ablösehilfe eine Schwächungslinie in der Schutzfolie oder ein nicht mit dem übrigen Teil der Schutzfolie verbundener Teil der Schutzfolie ist.

7. Applikationssystem nach einem der Ansprüche 1 bis 6, **dadurch gekennzeichnet, dass** die Schutzfolie des wirkstoffhaltigen Pflasters (3) bzw. die Schutzfolie des Wirkstoffabgaberegulierungsmittels (6) aus unterschiedlichen Materialien besteht.

8. Applikationssystem nach einem der Ansprüche 1 bis 7, **dadurch gekennzeichnet, dass** bei einer Faltung über die Faltlinie (5) die beiden Teile des Streifens zumindest eine so unterschiedliche Länge aufweisen, dass der Teil des Streifens (2), der mit dem Wirkstoffabgaberegulierungsmittel lösbar verbunden ist, von der Faltlinie ausgehend zumindest die gesamte Fläche des Wirkstoffabgaberegulierungsmittels (6) bedeckt.

9. Applikationssystem nach einem der Ansprüche 1 bis 7, **dadurch gekennzeichnet, dass** die beiden Teile des Streifens (2) nach einer Faltung entlang der Faltlinie (5) eine identische Länge aufweisen.

10. Applikationssystem nach einem der Ansprüche 1 bis 9, **dadurch gekennzeichnet, dass** das teilbare wirkstoffundurchlässige Wirkstoffabgaberegulierungsmittel mehrschichtig ist und folgenden Schichtaufbau umfaßt:
a) eine außenliegende, lösbare Schutzschicht
b) eine Klebeschicht
c) eine Wirkstoffsperrschicht
d) eine weitere Klebeschicht, die mit dem Streifen (2) lösbar verbunden ist

11. Applikationssystem nach einem der Ansprüche 1 bis 10, **dadurch gekennzeichnet, dass** das teilbare Wirkstoffabgaberegulierungsmittel über Schwächungslinien, Perforationslinien oder Schnittlinien teilbar ist.

12. Applikationssystem nach einem der Ansprüche 1 bis 11, **dadurch gekennzeichnet, dass** das wirkstoffundurchlässige Wirkstoffabgaberegulierungsmktel so teilbar ist, dass jeweils 10 bis 90 % der wirkstoffhaltigen Fläche (4) des Pflasters bedeckbar sind.

13. Applikationssystem nach einem der Ansprüche 1 bis 11, **dadurch gekennzeichnet, dass** die Größe des wirkstoffundurchlässigen Wirkstoffabgaberegulierungsmittel jeweils 10 bis 90 % der wirkstoffhaltigen Fläche (4) des Pflasters entspricht.

14. Applikationssystem nach einem Ansprüche 1 bis 11, **dadurch gekennzeichnet, dass** die Größe des die Wirkstoffabgabe retardierendes Wirkstoffabgaberegulierungsmittels jeweils 10 bis 100 % der wirkstoffhaltigen Fläche (4) des Pflasters entspricht.

15. Applikationssystem nach einem der Ansprüche 1 bis 14, **dadurch gekennzeichnet, dass** das Pflaster (3) ein wirkstoffhaltiges Reservoir oder eine wirkstoffhaltige Matrix aufweist

## Claims

1. An application system (1) for an active ingredient release system, comprising a film-form strip (2), an active ingredient-containing plaster (3) bonded detachably thereto and a divisible active ingredient release regulator (6), likewise bonded detachably thereto and separate from the active ingredient-containing plaster, the film-form strip (2) comprising a fold line (5) corresponding to its width and having a width which corresponds at least to the width of the active ingredient-containing plaster (3) and to a width optionally extending therebeyond of a peelable protective film used to cover the adhesive surface of the active ingredient-containing plaster (3), and the length of the strip (2) being a multiple of the length of the plaster (3), and the active ingredient release regulator (6) being permanently positionable after removal of the protective film peelable therefrom on the active ingredient-containing surface (4) of the plaster (3) after removal of its protective film by folding the strip (2) back together along the fold line (5) and the plaster provided with the active ingredient release regulator being applicable directly to the skin with removal of the strip (2), the active ingredient release regulator (6) having predetermined separating options by means of which it is divisible and the active ingredient release regulator (6) being impermeable to active ingredient or delaying active ingredient release and having the following layer structure:
a') an outer, detachable protective layer
b') an adhesive layer optionally delaying active ingredient release
c') optionally a layer optionally further delaying active ingredient release
d') and a further adhesive layer bonded detachably to the strip (2).

2. An application system according to claim 1, **characterised in that** the strip consists of a preferably transparent plastics film.

3. An application system according to claim 1 or claim 2, **characterised in that** at least the surface of the strip (2) to which the active ingredient release regulator is attached has abherent properties.

4. An application system according to any one of claims 1 to 3, **characterised in that** the active ingredient-containing plaster and the active ingredient release regulator are positioned on the same surface of the strip and in each case at such a distance from the fold line (5) that, on folding along the fold line (5) of the strip, the active ingredient release regulator may be positioned on the active ingredient-containing area of the plaster (4).

5. An application system according to any one of claims 1 to 4, **characterised in that** both the active ingredient release regulator (6) and the active ingredient-containing plaster (3) are covered with an outer, peelable protective film, which preferably comprises a peel aid.

6. An application system according to claim 5, **characterised in that** the peel aid is a line of weakness in the protective film or a part of the protective film not joined to the rest of the protective film.

7. An application system according to any one of claims 1 to 6, **characterised in that** the protective film of the active ingredient-containing plaster (3) and the protective film of the active ingredient release regulator (6) consist of different materials.

8. An application system according to any one of claims 1 to 7, **characterised in that**, upon folding by means of the fold line (5), the two parts of the strip have at least such differing lengths that, starting from the fold line, the part of the strip (2) which is bonded detachably to the active ingredient release regulator covers at least the entire surface area of the active ingredient release regulator (6).

9. An application system according to any one of claims 1 to 7, **characterised in that** the two parts of the strip (2) are of identical length after folding along the fold line (5).

10. An application system according to any one of claims 1 to 9, **characterised in that** the divisible, active ingredient-impermeable active ingredient release regulator is multilayered and has the following layer structure:
a) an outer, detachable protective layer
b) an adhesive layer
c) an active ingredient barrier layer
d) a further adhesive layer, which is bonded detachably to the strip (2).

11. An application system according to any one of claims 1 to 10, **characterised in that** the divisible active ingredient release regulator is divisible by way of lines of weakness, lines of perforations or cut lines.

12. An application system according to any one of claims 1 to 11, **characterised in that** the active ingredient-impermeable active ingredient release regulator is divisible in such a way that in each case 10 to 90% of the active ingredient-containing surface area (4) of the plaster may be covered.

13. An application system according to any one of claims 1 to 11, **characterised in that** the size of the active ingredient-impermeable active ingredient release regulator corresponds in each case to 10 to 90% of the active ingredient-containing surface area (4) of the plaster.

14. An application system according to any one of claims 1 to 11, **characterised in that** the size of the active ingredient release-delaying active ingredient release regulator corresponds in each case to 10 to 100% of the active ingredient-containing surface area (4) of the plaster.

15. An application system according to any one of claims 1 to 14, **characterised in that** the plaster (3) comprises an active ingredient-containing reservoir or an active ingredient-containing matrix.

## Revendications

1. Système d'application (1) pour un système de libération de substances actives comprenant une bande en forme de film (2), un pansement (3) contenant des substances actives assemblé de manière amovible avec celle-ci et un agent de régulation de la libération des substances actives (6) sécable, également assemblé de manière amovible avec celle-ci, séparé du pansement contenant des substances actives, la bande en forme de film (2) présentant une ligne de pliage (5) correspondant à sa largeur et présentant une largeur qui correspond au moins à la largeur du pansement (3) contenant des substances actives et à une largeur dépassant le cas échéant de celle-ci d'une feuille de protection amovible, utilisée pour recouvrir la surface adhésive du pansement (3) contenant des substances actives, et la longueur de la bande (2) étant un multiple de la longueur du pansement (3), et l'agent de régulation de la libération des substances actives (6) pouvant être positionné durablement, après élimination de la feuille de protection amovible de celui-ci sur la surface (4) contenant des substances actives du pansement (3) après l'élimination de sa feuille de protection, en repliant la bande (2) le long de la ligne de pliage (5) et le pansement, pourvu de l'agent de régulation de la libération des substances actives, pouvant être appliqué directement sur la peau en éliminant la bande (2), l'agent de régulation de la libération des substances actives (6) présentant des possibilités de séparation prédéfinies, via lesquelles il est sécable et l'agent de régulation de la libération des substances actives (6) étant un agent imperméable aux substances actives ou un agent retardant la libération des substances actives, qui présentent la structure à couches suivante :
a') une couche de protection amovible externe,
b') une couche adhésive retardant le cas échéant la libération des substances actives
c') le cas échéant une couche retardant le cas échéant davantage la libération des substances actives
d') et une autre couche adhésive assemblée de manière amovible avec la bande (2).

2. Système d'application selon la revendication 1, **caractérisé en ce que** la bande est constituée d'une feuille de matériau synthétique de préférence transparente.

3. Système d'application selon la revendication 1 ou 2, **caractérisé en ce qu'**au moins la surface de la bande (2), sur laquelle l'agent de régulation de la libération des substances actives est fixé, présente des propriétés antiadhésives.

4. Système d'application selon l'une quelconque des revendications 1 à 3, **caractérisé en ce que** le pansement contenant des substances actives et l'agent de régulation de la libération des substances actives sont positionnés sur la même surface de la bande et à chaque fois à une distance telle de la ligne de pliage (5) que lors d'un pliage le long de la ligne de pliage (5) de la bande, l'agent de régulation de la libération des substances actives peut être positionné sur la surface contenant des substances actives du pansement (4).

5. Système d'application selon l'une quelconque des revendications 1 à 4, **caractérisé en ce que** tant l'agent de régulation de la libération des substances actives (6) que le pansement (3) contenant des substances actives sont recouverts d'une feuille de protection amovible externe, qui présente de préférence une aide au détachement.

6. Système d'application selon la revendication 5, **caractérisé en ce que** l'aide au détachement est une ligne d'affaiblissement dans la feuille de protection ou une partie de la feuille de protection qui n'est pas assemblée avec le reste de la feuille de protection.

7. Système d'application selon l'une quelconque des revendications 1 à 6, **caractérisé en ce que** la feuille de protection du pansement (3) contenant des substances actives ou, selon le cas, la feuille de protection de l'agent de régulation de la libération des substances actives (6) est constituée de différents matériaux.

8. Système d'application selon l'une quelconque des revendications 1 à 7, **caractérisé en ce que** lors d'un pliage le long de la ligne de pliage (5), les deux parties de la bande présentent au moins une longueur différente telle que la partie de la bande (2) qui est assemblée de manière amovible avec l'agent de régulation de la libération des substances actives, partant de la ligne de pliage, recouvre au moins la surface totale de l'agent de régulation de la libération des substances actives (6).

9. Système d'application selon l'une quelconque des revendications 1 à 7, **caractérisé en ce que** les deux parties de la bande (2) après pliage le long de la ligne de pliage (5) présentent une longueur identique.

10. Système d'application selon l'une quelconque des revendications 1 à 9, **caractérisé en ce que** l'agent de régulation de la libération des substances actives, sécable, imperméable aux substances actives est à plusieurs couches et comprend la structure à couches suivante :
a) une couche de protection amovible externe,
b) une couche adhésive
c) une couche de blocage des substances actives
d) une autre couche adhésive qui est assemblée de manière amovible avec la bande (2).

11. Système d'application selon l'une quelconque des revendications 1 à 10, **caractérisé en ce que** l'agent de régulation de la libération des substances actives, sécable, peut être divisé via des lignes d'affaiblissement, des lignes de perforations ou des lignes de découpe.

12. Système d'application selon l'une quelconque des revendications 1 à 11, **caractérisé en ce que** le système de régulation de la libération des substances actives, imperméable aux substances actives peut être divisé de manière telle qu'à chaque fois 10 à 90% de la surface (4) contenant des substances actives du pansement peuvent être recouverts.

13. Système d'application selon l'une quelconque des revendications 1 à 11, **caractérisé en ce que** la taille de l'agent de régulation de la libération des substances actives imperméable aux substances actives correspond à chaque fois à 10 jusqu'à 90% de la surface (4) contenant des substances actives du pansement.

14. Système d'application selon l'une quelconque des revendications 1 à 11, **caractérisé en ce que** la taille de l'agent de régulation de la libération des substances actives retardant la libération des substances actives correspond à chaque fois à 10 jusqu'à 100% de la surface (4) contenant des substances actives du pansement.

15. Système d'application selon l'une quelconque des revendications 1 à 14, **caractérisé en ce que** le pansement (3) présente un réservoir contenant des substances actives ou une matrice contenant des substances actives.
